(19) Europäisches Patentamt
European Patent Office
Office européen des brevets

(11) Veröffentlichungsnummer: **0 376 890**
**A2**

# (12) EUROPÄISCHE PATENTANMELDUNG

(21) Anmeldenummer: **89810975.6**

(22) Anmeldetag: **21.12.89**

(51) Int. Cl.5: **C07F 7/18, C08G 18/10, C08G 18/38, C08K 5/54**

Claims for the following Contracting State: ES.

(30) Priorität: **29.12.88 CH 4845/88**

(43) Veröffentlichungstag der Anmeldung: **04.07.90 Patentblatt 90/27**

(84) Benannte Vertragsstaaten: **AT BE CH DE ES FR GB IT LI NL SE**

(71) Anmelder: **CIBA-GEIGY AG**
**Klybeckstrasse 141**
**CH-4002 Basel(CH)**

(72) Erfinder: **Parrinello, Giovanni, Dr.**
**Tervuursesteenweg 87**
**B-1982 Duisburg(BE)**
Erfinder: **Mülhaupt, Rolf, Prof.Dr.**
**Chemin de la Prairie 2**
**CH-1723 Marly(CH)**
Erfinder: **Simon, Hubert, Dr.**
**Rue A. Böhringer 42**
**F-68100 Mulhouse(FR)**

## (54) Silangruppenhaltige Oxazolidine.

(57) Verbindungen der allgemeinen Formel I

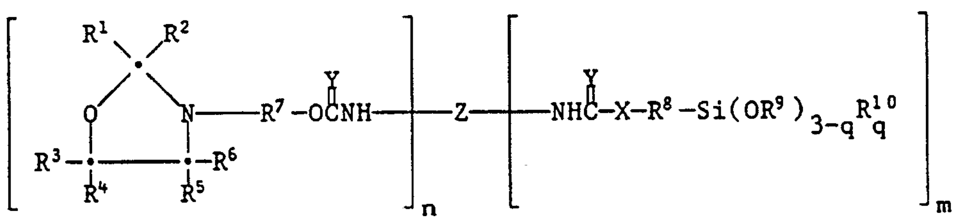

(I)

worin
$R^1$ Wasserstoff, $C_1$-$C_{12}$-Alkyl, $C_5$-$C_7$-Cycloalkyl, Phenyl oder Benzyl ist und
$R^2$ Wasserstoff oder $C_1$-$C_4$-Alkyl bedeutet, oder
$R^1$ und $R^2$ zusammen mit dem C-Atom, an welches sie gebunden sind, einen 5- oder 6-gliedrigen Ring bilden, und $R^3$, $R^4$, $R^5$ und $R^6$ gleich oder verschieden sind und Wasserstoff, $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder eine Gruppe der Formel $-CH_2OR^{11}$ bedeuten, wobei $R^{11}$ $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder -C(O)-$R^{12}$ ist, und $R^{12}$ $C_1$-$C_{12}$-Alkyl bedeutet, und
$R^7$ $C_1$-$C_4$-Alkylen, $R^8$ $C_1$-$C_8$-Alkylen, $R^9$ $C_1$-$C_4$-Alkyl oder zwei Reste $R^9$ zusammen $C_1$-$C_4$-Alkylen, sowie $R^{10}$ $C_1$-$C_4$-Alkyl oder Phenyl darstellen, wobei q die Werte 0 bis 2 bedeutet, ferner X -S- oder -NH- ist, Y = O oder = S ist, und Z ein organischer Rest ist, der sich von einem Polyisocyanat oder einem Polyisothiocyanat mit mindestens 3 NCO- resp. NCS-Gruppen ableitet, und weiter n einen Wert ≥ 1 und m einen Wert ≥ 1 bedeuten, mit der Massgabe, dass n + m ≥ 3 ist, eignen sich als Haftvermittler, insbesondere für feuchtigkeitshärtende Polyurethanharze.

EP 0 376 890 A2

## Silangruppenhaltige Oxazolidine

Die vorliegende Erfindung betrifft neue silangruppenhaltige 1,3-Oxazolidine, deren Herstellung, deren Verwendung als Haftvermittler, sowie 1- oder 2-Komponenten Polyurethanharze, welche diese Haftvermittler enthalten und als Klebstoffe, Dichtungsmassen, Oberflächenbeschichtungen oder Isolierstoffe eingesetzt werden.

1,3-Oxazolidine sind z.B. in der US-PS 3,743,626 als Härtungsmittel für feuchtigkeitshärtende Polyurethan-Präpolymere beschrieben worden.

Solche Präpolymere sind als Komponenten von Klebstoffen, Dichtungsstoffen, Anstrichstoffen oder Isolierstoffen bekannt. Die Härtbarkeit dieser Präpolymere beruht auf ihrem Gehalt an freien Isocyanatgruppen, wodurch unter Feuchtigkeitseinwirkung die Aushärtung eintritt. Die Haftung der ausgehärteten Polyurethane auf Glas oder Metall ist in vielen technischen Anwendungen unbefriedigend, was zum Einsatz von Grundiermitteln ("Primer") führte. Damit wird ein guter Verbund zwischen Polyurethan und Glas oder Metall erreicht, der auch durch hohe Feuchtigkeit, erhöhte Temperaturen und hohe mechanische Belastung wenig beeinträchtigt wird. Als Primer haben sich z.B. Aminoalkyl-alkoxysilane bewährt (vgl. Plueddemann et al. "Silan coupling agents", Plenum Press, NY [1982]). Allerdings sind die wirksamsten Aminosilan-Haftvermittler als eingebaute Haftvermittler in feuchtigkeitshärtenden Polyurethanen unmodifiziert nicht zu verwenden, da die Aminogruppen mit Isocyanatgruppen abreagieren. Daher sind in der DE-OS 3,414,877 Ketimine und Aldimine von Aminoalkylsilanen beschrieben worden, welche Polyurethan-Klebstoffen zugesetzt werden können ohne deren Lagerstabilität zu beeinträchtigen.

Weiter sind aus der US-PS 4,772,716 silangruppenhaltige 1,3-Oxazolidine als Haftvermittler für feuchtigkeitshärtende Epoxid- oder Polyurethanharze bekannt.

Niederfunktionelle Oxazolidin Haftvermittler für Polyurethane bewirken gute Haftung auf Glas ohne Primervorbehandlung doch beobachtet man oft eine reduzierte Härtungsgeschwindigkeit.

Es wurde nun eine Klasse von Verbindungen gefunden, die 1- oder 2-Komponenten-Polyurethanharz-Klebstoffen, -Dichtungsmassen, -Lacken sowie -Isolierstoffen zugesetzt werden, wobei eine signifikant erhöhte Haftung auf Glas, Metall, lackiertem Stahl sowie Kunststoffen erzielt wird, und gleichzeitig die Härtungsgeschwindigkeit nicht beeinträchtigt oder sogar erhöht wird.

Die vorliegende Erfindung betrifft Verbindungen der allgemeinen Formel I

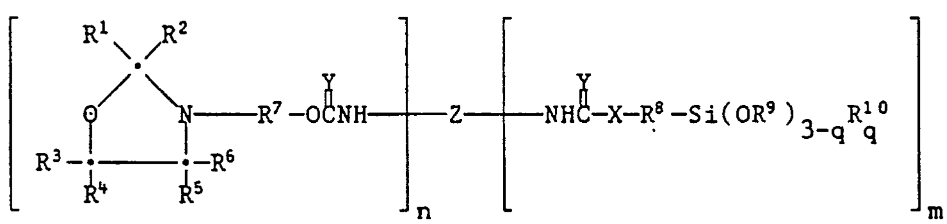

(I)

worin

$R^1$ Wasserstoff, $C_1$-$C_{12}$-Alkyl, $C_5$-$C_7$-Cycloalkyl, Phenyl oder Benzyl ist und

$R^2$ Wasserstoff oder $C_1$-$C_4$-Alkyl bedeutet, oder

$R^1$ und $R^2$ zusammen mit dem C-Atom, an welches sie gebunden sind, einen 5- oder 6-gliedrigen Ring bilden, und $R^3$, $R^4$, $R^5$ und $R^6$ gleich oder verschieden sind und Wasserstoff, $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder eine Gruppe der Formel $-CH_2OR^{11}$ bedeuten, wobei $R^{11}$ $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder -C(O)-$R^{12}$ ist, und $R^{12}$ $C_1$-$C_{12}$-Alkyl bedeutet, und

$R^7$ $C_1$-$C_4$-Alkylen, $R^8$ $C_1$-$C_8$-Alkylen, $R^9$ $C_1$-$C_4$-Alkyl oder zwei Reste $R^9$ zusammen $C_1$-$C_4$-Alkylen, sowie $R^{10}$ $C_1$-$C_4$-Alkyl oder Phenyl darstellen, wobei q die Werte 0 bis 2 bedeutet, ferner X -S- oder -NH- ist, Y = O oder = S ist, und Z ein organischer Rest ist, der sich von einem Polyisocyanat oder einem Polyisothiocyanat mit mindestens 3 NCO- resp. NCS-Gruppen ableitet, und weiter n einen Wert $\geqq 1$ und m einen Wert $\geqq 1$ bedeuten, mit der Massgabe, dass $n + m \geqq 3$ ist.

Sind $R^1$, $R^3$, $R^4$, $R^5$, $R^6$, $R^{11}$ und $R^{12}$ $C_1$-$C_{12}$-Alkyl, bevorzugt $C_1$-$C_4$-Alkyl, so handelt es sich beispielsweise um Methyl, Ethyl, n-Propyl, Isopropyl, n-Butyl, Isobutyl, t.-Butyl, n-Pentyl, n-Hexyl, geradkettiges oder verzweigtes Octyl, Nonyl, Decyl, Undecyl oder Dodecyl.

Die bevorzugte Bedeutung von $R^3$, $R^4$, $R^5$ und $R^6$ als Alkyl ist Methyl.

Besonders bevorzugt sind Verbindungen der Formel I, worin $R^3$, $R^4$, $R^5$ und $R^6$ Wasserstoff, $C_1$-$C_4$-

Alkyl, und höchstens zwei dieser Reste Phenoxymethyl sind und insbesondere Verbindungen, worin $R^3$ und $R^6$ Wasserstoff und $R^4$ und $R^5$ Methyl sind.

Kommen die Symbole $R^3$, $R^4$, $R^5$ und $R^6$ in Verbindungen mit n = 2 zweimal vor, so haben diese beiden Reste bevorzugt jeweils die gleiche Bedeutung.

Bedeuten $R^2$, $R^9$ und $R^{10}$ $C_1$-$C_4$-Alkyl, so können sie beispielsweise Methyl, Ethyl, n-Propyl, Isopropyl, n-Butyl, Isobutyl oder t.-Butyl darstellen.

Bedeuten $R^7$ und $R^9$ bzw. $R^8$ $C_1$-$C_4$ bzw $C_1$-$C_8$-Alkylen, so handelt es sich dabei um geradkettiges oder verzweigtes Alkylen, wobei das geradkettige Alkylen bevorzugt ist. Beispielsweise handelt es sich um Methylen, Ethylen, Propylen, Trimethylen, Tetramethylen, 2-Methyl-1,3-trimethylen und für $R^8$ zusätzlich um Pentamethylen, 2-Methyl-1,4-tetramethylen, 3-Propyl-1,3-trimethylen, 1,6-Hexamethylen, 1,7-Heptamethylen, 1,8-Octamethylen oder 2-Ethyl-1,2-hexamethylen. Bevorzugt bedeuten $R^7$ und $R^9$ Ethylen, sowie $R^8$ $C_1$-$C_4$-Alkylen, insbesondere Trimethylen oder Ethylen.

Bedeutet $R^1$ $C_5$-$C_7$-Cycloalkyl, so handelt es sich vorzugsweise um Cyclopentyl oder Cyclohexyl.

In bevorzugten Verbindungen sind $R^1$ und $R^2$ Wasserstoff oder $C_1$-$C_4$-Alkyl, und insbesondere ist einer der Reste $R^1$ und $R^2$ Wasserstoff und der andere $C_1$-$C_4$-Alkyl, vorzugsweise Isopropyl oder tert.-Butyl.

Sind $R^3$, $R^4$, $R^5$, $R^6$ und $R^{11}$ substituiertes Phenyl, so kommen z.B. die Reste o-, m- oder p-Methylphenyl, 2,4-Dimethylphenyl, 2,4,6-Trimethylphenyl, p-Ethylphenyl, p-t.-Butylphenyl, 2-Chlorphenyl oder 4-Methoxyphenyl in Frage.

Der Rest Z leitet sich von einem Polyisocyanat oder Polyisothiocyanat mit mindestens 3 NCO- resp. NCS-Gruppen ab. Diese erfindungsgemässe NCO-resp. NCS-Funktionalität ≧ 3 des Polyisocyanats bzw. Polyisothiocyanats wird dadurch erreicht, dass beispielsweise Polyamine wie z.B. aminoterminierte Polyetherpolyole durch Phosgenierung oder Thiophosgenierung zu Polyisocyanaten bzw. Polyisothiocyanaten mit einer Funktionalität ≧ 3 umgesetzt werden. Die so erhältlichen Polyisocyanate oder Polyisothiocyanate können entweder direkt eingesetzt werden oder zuerst mit Diolen, Polyolen, Dimercaptanen, Diaminen oder Polyaminen zu NCO- resp. NCS-terminierten Präpolymeren umgesetzt werden. Ebenfalls können die auf nachstehende Weise erhältlichen Polyisocyanate so umgesetzt werden.

Eine weitere Möglichkeit, Polyisocyanate mit einer NCO-Funktionalität ≧ 3 herzustellen, besteht in der Oligomerisierung von Diisocyanaten. So können beispielsweise Diisocyanate wie z.B. Hexamethylendiisocyanat durch partielle Hydrolyse zu biuretgruppenhaltigen Produkten (wie z.B. Desmodur® N100 von Bayer) umgesetzt werden.

Weiter können Diisocyanate wie z.B. Hexamethylendiisocyanat partiell trimerisiert werden, so dass höher funktionelle Polyisocyanate entstehen, welche Isocyanurat-Ringe enthalten wie z.B. Desmodur® N3200 von Bayer.

Eine Kettenverlängerung durch Umsetzung von Diisocyanaten mit polyfunktionellen H-aciden Verbindungen mit einer Funktionalität ≧ 3 wie z.B. Triole, Tetrole, Pentole, Triamine, Polyamine oder Polymercaptane führt ebenfalls zu Polyisocyanaten mit einer NCO-Funktionalität ≧ 3. Dabei ist das NCO/OH-Verhältnis > 1, vorzugsweise jedoch > 3:1, insbesondere > 10:1.

Als Diisocyanate eignen sich hier sowohl aromatische als auch aliphatische, heterocyclische, monocyclische und polycyclische, bifunktionelle Isocyanatverbindungen. Beispiele für solche Verbindungen sind Toluylen diisocyanat, Diphenylmethandiisocyanat, Naphthylendiisocyanat, Xylylendiisocyanat, Hexamethylendiisocyanat, Trimethylhexamethylendiisocyanat, Isophorondiisocyanat oder Dicyclohexylmethandiisocyanat.

Die Parameter m und n besitzen unabhängig voneinander die Werte 1 bis 49, bevorzugt 1 bis 9, besonders bevorzugt 1 bis 5 und ganz speziell bevorzugt 1, 2 und 3. Die Summe n + m beträgt im allgemeinen 3 bis 50, bevorzugt 3 bis 10, insbesondere 3 bis 6.

Der Rest Z weist vorzugsweise ein mittleres Molekulargewicht Mn < 10000, insbesondere Mn < 4000, auf.

Y stellt bevorzugt = O dar.

Bevorzugte Verbindungen entsprechen der Formel I, worin Z von einem aliphatischen, cycloaliphatischen, aliphatisch/aromatischen, aromatischen oder heterocyclischen Polyisocyanat oder Polyisothiocyanat mit ≧ 3 NCO-oder NCS-Gruppen abgeleitet ist, wobei dieser Rest Z gegebenenfalls eine oder mehrere Ester-, Ether-, Urethan-, Thiourethan-, Isocyanurat-, Harnstoff- oder Biuret-Funktionen enthalten kann.

Besonders bevorzugte Verbindungen entsprechen der Formel I, worin Z von einem aliphatischen oder gemischt aliphatisch/aromatischen Polyisocyanat mit ≧ 3 NCO-Gruppen abgeleitet ist, wobei dieser Rest Z gegebenenfalls gesamthaft eine oder zwei Ester-, Ether-, Urethan-, Thiourethan-, Isocyanurat-, Harnstoff-, oder Biuret-Funktionen enthält.

Besitzt Z in den Verbindungen der Formel I Ethersauerstoffe, so kann es sich um Monoether oder Oligoether, wie z.B. eine Gruppe der Formel $-(CH[CH_3]-CH_2-O)_y-$ oder $-(CH_2CH_2CH_2CH_2-O)_y$ handeln,

wobei y eine Zahl 1-80, vorzugsweise 1-20 bedeutet.

Sind im Rest Z in den Verbindungen der Formel I Carbamat- oder Thiocarbamat-Gruppen enthalten, so handelt es sich um Derivate, welche durch Umsetzung von Polyolen mit Isocyanat- bzw. Isothiocyanat-Gruppen ent haltenden Verbindungen erhältlich sind. Es sind auch Reste darunter zu verstehen, welche sowohl eine oder mehrere Urethangruppen als auch eine oder mehrere Thiourethangruppen enthalten, etwa solche welche ein Brückenglied der Formel

$-O\overset{O}{\overset{\|}{C}}-NH-P-NH-\overset{O}{\overset{\|}{C}}-O-$ bzw.

$-S-\overset{O}{\overset{\|}{C}}-NH-P-NH-\overset{O}{\overset{\|}{C}}-S-$ oder

$-S-\overset{O}{\overset{\|}{C}}-NH-P-NH-\overset{O}{\overset{\|}{C}}-O-$ enthalten, wobei P der Rest des Polyols bedeutet.

Als Polyole können z.B. auch OH-terminierte Polyether oder Polyester eingesetzt werden.

In bevorzugten Verbindungen der Formel I enthält der Rest Z zwei, in besonders bevorzugten Verbindungen eine Ester-, Carbamat-, Isocyanurat; Harnstoff- oder Biuret-Funktionen. Dabei stellen die Etherfunktionen eine gewisse Ausnahme dar, da sie, wie oben angezeigt wurde, in der Lage sind Oligoether-Brückenglieder zu bilden. Solche Verbindungen können daher bis zu 80, bevorzugt bis zu 20 Etherfunktionen enthalten.

Speziell bevorzugt werden Verbindungen der Formel I, worin $R^1$ $C_1$-$C_4$-Alkyl, insbesondere Isopropyl oder tert.-Butyl, $R^2$ Wasserstoff, $R^3$, $R^4$, $R^5$ und $R^6$ je Wasserstoff und $R^7$ Ethylen bedeuten und n 1 oder 2 ist.

Ebenfalls speziell bevorzugt werden Verbindungen der Formel I, worin X -S-, $R^8$ $C_1$-$C_4$-Alkylen, insbesondere Trimethylen, und $R^9$ $C_1$-$C_4$-Alkyl, insbesondere Methyl oder Ethyl, bedeuten und q den Wert 0 hat.

Ganz speziell bevorzugt wird die Verbindung der Formel I, worin $R^1$ Isopropyl, $R^2$, $R^3$, $R^4$, $R^5$ und $R^6$ je Wasserstoff, $R^7$ Ethylen, $R^8$ Trimethylen, $R^9$ Methyl, Y = O, X -S- und Z

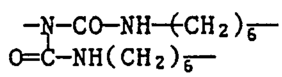

bedeuten und m den Wert 2, n den Wert 1 und q den Wert 0 besitzen.

Beispiele von Verbindungen der Formel I sind:

| n | $R^1$ | $R^2$ | $R^3$ | $R^6$ | $R^7$ | m | X | Y | $R^8$ | $R^9$ | q | $R^{10}$ | Z |
|---|---|---|---|---|---|---|---|---|---|---|---|---|---|
| 1 | H | 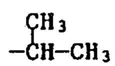 | 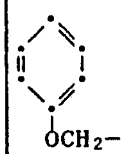 | H | 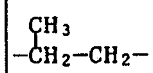 | 2 | S | O | $-(CH_2)_3-$ | $-CH_3$ | 0 | - | 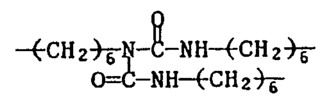 |
| 2 | $-CH_2-CH_2-CH_2-CH_2-$ | | $-CH_3$ | $-CH_3$ | $-CH_2-CH_2-$ | 2 | S | O | $-(CH_2)_3-$ | $-CH_2-CH_3$ | 0 | - | 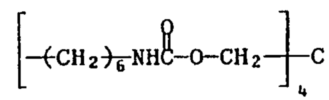 |
| 2 | H | 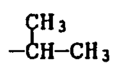 | H | H | $-CH_2-CH_2-$ | 1 | S | O | $-(CH_2)_3-$ | $-CH_3$ | 0 | - | 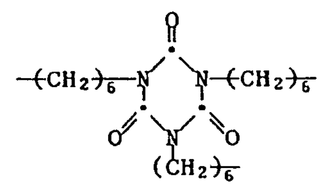 |
| 1 | H | $-CH_3$ | $-CH_3$ | H | 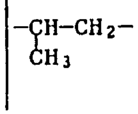 | 2 | NH | O | $-(CH_2)_3-$ | $-CH_3$ | 1 | 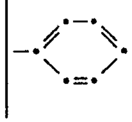 | 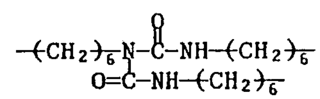 |

| n | $R^1$ | $R^2$ | $R^3$ | $R^6$ | $R^7$ | m | X | Y | $R^8$ | $R^9$ | q | $R^{10}$ | Z |
|---|---|---|---|---|---|---|---|---|---|---|---|---|---|
| 4 | H | $-CH(CH_3)-CH_3$ | H | H | $-CH_2-CH_2-$ | 4 | S | O | $-(CH_2)_3-$ | $-CH_3$ | O | - | 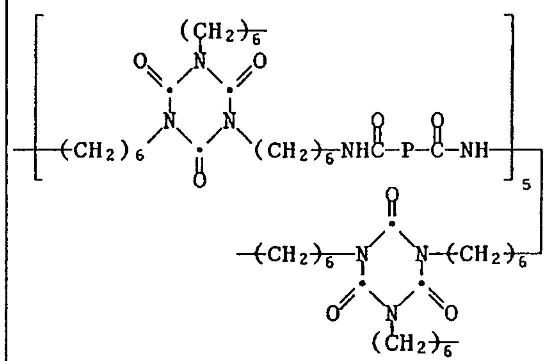 P: $-OCH_2-CH(CH_3)-CH_2-(OCH(CH_3)-CH_2)_{18}-O-$ |

Dabei sind Verbindungen mit $R^4$ bzw. $R^5$ in der Bedeutung von Wasserstoff gemeint.

Die Herstellung der Verbindungen der Formel I erfolgt auf an sich bekannte Weise und kann am einfachsten anhand der folgenden Reaktionsschematas wiedergegeben werden.

I. Oxazolidinteil

I.1.

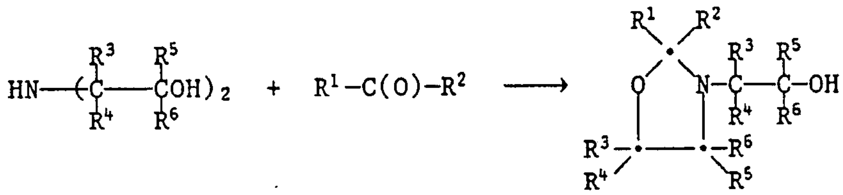

(A) (B) (C)

Diese Methode erfolgt nach der in der DE-OS 24 26 438 beschriebenen Weise. Die Edukte (A) und (B) sind bekannte Verbindungen, zum Teil im Handel erhältlich oder können auf einfache bekannte Weise hergestellt werden. Als Edukte (A) eignen sich besonders Bis(2-hydroxyethyl)-amin und Bis-(2-hydroxypropyl)-amin. Als Edukte (B) können z.B. die Carbonylverbindungen Formaldehyd, Acetaldehyd, Propionaldehyd, Butyraldehyd, Isobutyraldehyd, Benzaldehyd, Aceton, Methylbutylketon, Cyclopentanon oder Cyclohexanon in Frage kommen.

I.2.

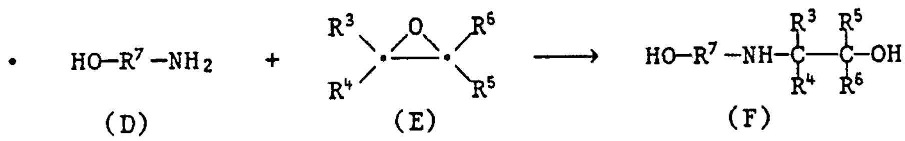

(D) (E) (F)

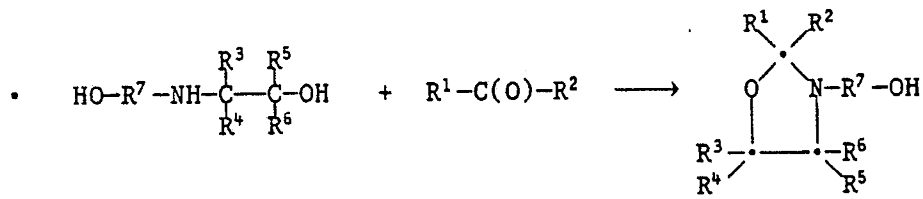

(F) (B) (G)

Diese Methode erfolgt nach der in der EP-A 96,768 beschriebenen Weise. Die Edukte (D), (E) und (B) sind bekannte Verbindungen, welche zum Teil handelsüblich sind oder auf bekannte Weise hergestellt werden können. Als Epoxidkomponente werden vorzugsweise die in der Epoxidharzchemie gebräuchlichen reaktiven Verdünner eingesetzt.

In der ersten Stufe werden die Komponenten (D) und (E) in ungefähr äquimolarer Menge bei einer Temperatur von ungefähr 60-130 °C umgesetzt. In der zweiten Stufe wird die Komponente (F) mit der carbonylverbindung (B) bei ungefähr 80-110 °C umgesetzt, wobei das entstehende Reaktionswasser azeotrop mit Hilfe eines Schleppmittels wie z.B. einem Benzinkohlenwasserstoff abgetrennt wird.

Die so hergestellten N-Hydroxyalkyl-oxazolidine (C) und (G) können in einer weiteren Stufe mit einem Polyisocyanat $Z(NCO)_{\geq 3}$ oder einem Polyisothiocyanat $Z(NCS)_{\geq 3}$ umgesetzt werden.

II. Silanteil

Die erfindungsgemäss verwendeten Amino- oder Mercapto-alkoxysilane sind bekannte Verbindungen. Sie sind zum Teil im Handel erhältlich oder können nach bekannten Methoden hergestellt werden. Verbindungen dieser Art sind in ausführlicher Weise in "Silane Coupling Agents" von E.P. Plueddemann, Plenum Press, New York, 1982, beschrieben.

III. Polyisocyanat $Z(NCO)_{\geq 3}$ oder Polyisothiocyanat $Z(NCO)_{\geq 3}$

Die Herstellung dieser Komponenten erfolgt nach literaturbekannten Methoden wie sie z.B. in den US-PS 3,492,330; GB-PS 994,890; DE-PS 1,022,789; DE-PS 1,222,067; DE-PS 1,027,394; DE-OS 1,929,034; DE-OS 2,004,048; US-PS 3,394,164; DE-PS 1,101,394; GB-PS 889,050; BE-PS 723,640; GB-PS 956,474; GB-PS 1,072,956; US-PS 3,567,763 oder DE-PS 1,231,688 beschrieben werden.

Die Polyisothiocyanate können analog hergestellt werden. Anstelle von Diisocyanaten werden die entsprechenden Diisothiocyanaten als Edukte eingesetzt. Aliphatische Edukte können nach den in der US-PS 3,787,472 und aromatische Edukte nach der in "Org. Syntheses"; Collective Volume 1, S. 447 John Wiley, New York, (1948) beschriebenen Methoden hergestellt werden.

IV. Umsetzung der Polyisocyanate gemäss III. mit den Oxazolidinen (C) und (G) sowie mit den Silanen gemäss II. zu den Verbindungen der Formel I.

Die Umsetzung der Polyisocyanate resp. Polyisothiocyanate mit den andern beiden Komponenten kann nacheinander oder miteinander erfolgen. Bei stufenweiser Umsetzung kann zuerst die Oxazolidinverbindung (C) resp. (G) mit dem Polyisocyanat resp. Polyisothiocyanat umgesetzt werden und anschliessend das Addukt mit dem Alkoxysilan oder umgekehrt. Dabei ist es auch möglich verschiedene Oxazolidin- resp. Silan-Komponenten an das Polyisocyanat resp. -thiocyanat zu addieren, wobei es möglich ist, die verschiedenen Komponenten alternierend umzusetzen d.h. z.B. zuerst Addition eines Silans, dann Oxazolidinaddition und schliesslich Addition des zweiten Silans.

Die Umsetzung erfolgt in der Regel ohne Lösungsmittel, jedoch können bei Bedarf eine oder alle Komponenten durch ein geeignetes inertes Lösungsmittel verdünnt werden, um z.B. die Viskosität den Erfordernissen anzupassen.

Die Addition selbst erfolgt bei Temperaturen zwischen 15 °C und 200 °C, bevorzugt aber bei Temperaturen zwischen 30 °C und 140 °C.

Der Reaktionsverlauf kann durch Infrarotspektroskopie oder Titration verfolgt werden.

Bei den Additionsreaktionen können auch Katalysatoren der an sich bekannten Art mitverwendet werden, wie z.B. tertiäre Amine wie beispielsweise Triethylamin, N-Methyl-morpholin, N,N,N′,N′-Tetramethyl-ethylendiamin oder 1,4-Diaza-bicyclo-(2,2,2)-octan. Auch organische Metallverbindungen, insbesondere organische Zinnverbindungen, können als Katalysatoren verwendet werden.

Beispiele für organische Zinnverbindungen sind Zinn(II)-salze von Carbonsäuren, wie z.B. Zinn(II)-acetat, Zinn(II)-octoat und Zinn( II)-laurat, oder die Dialkylzinnsalze von Carbonsäuren, wie z.B. Dibutylzinndiacetat, Dibutylzinn-dilaurat oder Dioctyl-zinndiacetat.

Die stöchiometrischen Verhältnisse bei der Addition der Oxazolidin- und Silan-Komponenten an die Polyisocyanate resp. -thiocyanate sind so gehalten, dass das Verhältnis von OH-Gruppen der Oxazolidine und der $NH_2$-resp. SH-Gruppen der Silane ungefähr äquimolar zu den NCO- resp. NCS-Gruppen der Polyisocyanate resp. -thiocyanate ist. Dabei kann das Addukt noch freie NCO- resp. NCS-Gruppen enthalten. Vorzugsweise ist jedoch keine freie NGO- resp. NCS-Gruppe vorhanden.

Mit dem stöchiometrischen Verhältnis der Edukte bei der Additionsreaktion lässt sich das Verhältnis Oxazolidinreste zu Silanreste in den erfindungsgemässen Verbindungen der Formel I steuern. Dazu werden vorzugsweise die Oxazolidin- resp. die Silanverbindung in getrennten Schritten mit dem Polyisocyanat resp. -thiocyanat umgesetzt. Die erste Stufe erfolgt in der Regel bei einem Verhältnis von OH- resp. $NH_2$- oder SH-Gruppen zu NCO-bzw. NCS-Gruppen von kleiner als 1. Das bevorzugte OH/NCO- resp. NCS-Verhältnis liegt zwischen 1:2 bis 1:6, insbesondere zwischen 1:3 und 1:5. Das bevorzugte Verhältnis von $NH_2$- resp. SH/NCO-resp. NCS-Gruppen liegt zwischen 2:3 und 1:5, insbesondere zwischen 2:3 und 1:2.

In der zweiten Stufe werden in der Regel die restlichen freien NCO- resp. NCS-Gruppen mit den OH-bzw. $NH_2$- oder SH-Gruppen abreagiert. Dazu ist das stöchiometrische Verhältnis der H-aciden Gruppen zu NCO- resp. NCS-Gruppen ≧ 1, bevorzugt 4:1 bis 1:1, insbesondere 2:1 bis 1:1.

Es ist jedoch auch möglich, in der zweiten Stufe die restlichen freien NCO- resp. NCS-Gruppen nur partiell abzureagieren. Dann gelten die gleichen stöchiometrischen Verhältnisse wie bei der ersten Additionsstufe. Ein solches Vorgehen wird bevorzugt angewendet, wenn zwei oder mehr verschiedene Oxazolidin- resp. Silanverbindungen addiert werden.

Die erfindungsgemässen Verbindungen können grundsätzlich in verschiedenen Substraten als Haftvermittler eingesetzt werden. Besonders wirkungsvoll ist ihr Einsatz in feuchtigkeitshärtenden Polyurethanharzen, welche als Klebstoffe, Dichtungsmassen, Lacke oder Isolierstoffe zur Anwendung kommen. Handelt es sich um Klebstoffe, so besitzen die erfindungsgemässen Verbindungen Eigenschaften, welche ihren Einsatz in Zweikomponenten-und ganz besonders in Einkomponentensystemen ermöglicht. Der Einsatz der erfindungsgemässen Verbindungen als eingebaute Haftvermittler in den genannten Substraten macht eine Vorbehandlung der zu verklebenden Oberflächen mit einem Primer überflüssig. Als Anwendungsbeispiele sei das Verkleben von Windschutzscheiben und Scheinwerfer im Automobilbau erwähnt. Verbindungen der Formel I mit n≧2 können ausserdem als feuchtigkeitsaktivierte Härter für die genannten Substrate eingesetzt werden. Ferner können Verbindungen der Formel I als Primer zur Substratvorbehandlung eingesetzt werden.

Handelt es sich beim Substrat um ein feuchtigkeitshärtendendes Polyurethan, so enthalten diese als Hauptbestandteil mehrfunktionelle Isocyanate und/oder Polyurethan-Präpolymere. Geeignet sind hier sowohl aromatische als auch aliphatische, monocyclische wie polycyclische, mehrfunktionelle Isocyanatverbindungen. So kann nach einer ersten Ausführungsform als aromatische Isocyanatverbindung Toluylendiisocyanat oder Diphenylmethandiisocyanat eingesetzt werden. Besonders geeignet ist technisches Diphenylmethandiisocyanat mit einem Gehalt an höherfunktionellen Diisocyanaten und einer Funktionalität an Isocyanatgruppen grösser als 2. Ein weiteres geeignetes aliphatisches Diisocyanat ist Xylylendiisocyanat. Darüber hinaus kann eine Vielzahl aliphatischer Isocyanate der Funktionalität 2 und höher eingesetzt werden. Beispiele sind hier Isophorondiisocyanat und Dicyclohexylmethandiisocyanat als cyclische aliphatische Diisocyanate. Weitere Beispiele sind aliphatische, geradkettige Diisocyanate, wie sie durch Phosgenisierung von Diaminen gewonnen werden, z.B. Tetramethylendiisocyanat oder Hexamethylendiisocyanat.

Nach einer bevorzugten Ausführungsform der Erfindung werden anstatt der mehrfunktionellen Isocyanatverbindungen Polyurethan-Präpolymere eingesetzt. Unter Präpolymeren werden hier die Addukte eines Ueberschusses mehrfunktioneller Isocyanate an mehrfunktionelle Alkohole, etwa die Umsetzungsprodukte eines der vorgenannten aromatischen oder aliphatischen Diisocyanate mit Ethylenglykol, Propylenglykol, Glycerin, Trimethylolpropan oder Pentaerythrit verstanden. Auch Umsetzungsprodukte von Diisocyanaten mit Polyetherpolyolen, z.B. Polyetherpolyolen auf Basis Polyethylenoxid oder auf Basis Polypropylenoxid können als Prepolymere verwendet werden. Bevorzugt sind Polyurethan-Präpolymere auf Basis von Polyetherpolyolen mit Molgewichten zwischen 200 und 10000, insbesondere 500 und 3000.Dem Polyurethan-Fachmann sind eine grosse Anzahl derartiger Polyetherpolyole bekannt; sie werden von zahlreichen Herstellern angeboten und über ihr Molekulargewicht (Zahlenmittel), das aus Endgruppenbestimmungen errechenbar ist, charakterisiert. Weitere geeignete Polyetherpolyole sind Polyetherpolyole auf Basis Polytetrahydrofuran.

Anstelle von Polyetherpolyole können auch Polyesterpolyole eingesetzt werden. Geeignete Polyesterpolyole sind Umsetzungsprodukte mehrfunktioneller Säuren mit mehrfunktionellen Alkoholen, beispielsweise Polyester auf Basis aliphatischer und/oder aromatischer Dicarbonsäure und mehrfunktioneller Alkohole der Funktionalität 2-4. So können Polyester aus Adipinsäure, Sebacinsäure, Phthalsäure, Hydrophthalsäure und/oder Trimellitsäure einerseits und Ethylenglykol, Propylenglykol, Neopentylglykol, Hexanglykol, Glycerin und/oder Trimethylolpropan andererseits eingesetzt werden. Geeignet sind insbesondere Polyesterpolyole mit Molekulargewicht (Zahlenmittel) zwischen 500 und 5000, insbesondere zwischen 600 und 2000. Weitere geeignete Polyesterpolyole sind die Umsetzungsprodukte von Caprolacton mit Alkoholen der Funktionalität von 2-4, so beispielsweise das Additionsprodukt von 1-5 Mol Caprolacton an 1 Mol Ethylenglykol, Propylenglykol, Glycerin und/oder Trimethylpropan.

Eine weitere geeignete Klasse mehrfunktioneller Alkohole sind Polybutadienole. Hierbei handelt es sich um Oligomere auf Basis Butadien, die als Endgruppen OH-Gruppen aufweisen. Geeignet sind hier Produkte im Molekulargewichtsbereich 200-4000, insbesondere 500-3000. Ferner sind Siloxanpräpolymere, vorzugsweise in Kombination mit andern Präpolymeren, geeignet.

Bei der Herstellung der Polyurethan-Präpolymeren ist das Verhältnis von OH-Gruppen der Alkoholkomponente zu Isocyanatgruppen von Bedeutung. Dieses liegt im allgemeinen zwischen 1:2 und 1:10. Dabei werden mit höheren Isocyanatüberschüssen eher niedrigviskose Polyurethan-Präpolymere erhalten, während niedere Isocyanatüberschüsse hochviskose, meist nur noch spachtelbare Zubereitungen liefern.

Es ist dem Polyurethan-Fachmann bekannt, dass die Vernetzungsdichte und damit die Härte der Polyurethane mit der Funktionalität der Isocyanatkomponente oder auch des Polyols zunimmt. Es sei hier

auf die allgemeine Fachliteratur verwiesen, z.B. auf die Monographie von Saunders und Frisch "Polyurethanes, Chemistry and Technology", Band XVI der Serie High Polymers "Intescience Publishers" New York-London, Teil I (1962) und Teil II (1964).

Die erfindungsgemässen Polyurethanzubereitungen können weiterhin verschiedene Hilfsstoffe enthalten. Verwendet werden können hier z.B. Füllstoffe. Als Füllstoffe geeignet sind gegenüber Isocyanaten nichtreaktive, anorganische Verbindungen wie z.B. Kreide oder Kalkmehl, gefällte und/oder pyrogene Kieselsäuren, Zeolithe, Bentonite, gemahlene Mineralien sowie andere dem auf dem Arbeitsgebiet tätigen Fachmann bekannte anorganische Füllstoffe, insbesondere Faserkurzschnitte und anderes. Für manche Anwendungen sind Füllstoffe bevorzugt, die den Zubereitungen Thixotropie verleihen, so beispielsweise quellbare Kunststoffe insbesondere PVC.

Ausser den genannten Verbindungen können die erfindungsgemässen Polyurethanzubereitungen noch weitere Hilfsstoffe, beispielsweise Lösungsmittel enthalten. Geeinget sind Lösungsmittel, die ihrerseits nicht mit Isocyanatgruppen reagieren, so z.B. halogenierte Kohlenwasserstoffe, Ester, Ketone, aromatische Kohlenwasserstoffe u.a.. Auch Weichmacher, Flammschutzmittel, Verzögerer, Farbstoffe und Alterungsschutzmittel, wie sie in Polyurethan-Klebstoffen und Dichtungsmassen bekannt sind, können mit eingearbeitet werden.

Für manche Anwendung ist es wünschenswert, den erfindungsgemässen Polyurethanzubereitungen Schaumstabilisatoren zuzusetzen. Als Schaumstabilisatoren können sogenannte Silikotenside verwendet werden. Es sind dies Blockcopolymere, die aus einem Polysiloxanblock und einem oder mehreren Polyoxyethylen- und/oder Polyoxypropylenblöcken aufgebaut sind. Die erfindungsgemässen Polyurethanzubereitungen können weiterhin flammhemmende und weichmachende Zusätze enthalten. Gängig sind Phosphor-und/oder Halogenatome enthaltende Verbindungen, wie Tricresylphosphat, Diphenylcresylphosphat, Tris-2-chlor-ethylphosphat, Tris-2-chlorpropylphosphat und Tris-2,3-dibrompropylphosphat. Zusätzlich können Flamm schutzmittel verwendet werden, z.B. Chlorparaffine, Halogenphosphide, Ammoniumphosphat und halogen- und phosphorhaltige Harze. Als weitere Zusätze sind für manche Anwendungen Weichmacher von Bedeutung. Geeignet sind hier beispielsweise Ester der Phthalsäure oder Ester langkettiger Dicarbonsäuren, beispielsweise Sebacinsäure- oder Azelainsäureester. Auch sogenannte Epoxidweichmacher, z.B. epoxydierte Fettsäurederivate können hier eingesetzt werden.

Weitere mögliche Zusätze sind basische Beschleuniger. Basische Beschleuniger sind beispielsweise tertiäre Basen wie Bis-(N,N′-dimethylamino)-diethylether, Dimethylamino-cyclohexan, N,N-Dimethyl-benzylamin, N-Methylmorpholin sowie die Umsetzungsprodukte von Dialkyl-($\beta$-hydroxyethyl)-amin mit Monoisocyanaten und Veresterungsprodukte von Dialkyl-($\beta$-hydroxyethyl)-amin und Dicarbonsäuren. Ein weiterer wichtiger Beschleuniger ist das 1,4-Diamino-bicyclo-(2.2.2)-octan. Ferner können nicht-basische Substanzen als Beschleuniger verwendet werden. Hier seien Metallverbindungen genannt, beispielsweise Eisenacetylacetonat sowie Zinn-(II)-2-ethylhexoat, Dibutylzinndilaurat oder Molybdänglykolat.

Polyurethan-Präpolymeren werden die Verbindungen der Formel I in Mengen von 0,1-20 Gew.%, bevorzugt 0,5-5 Gew.%, insbesondere 0,5-2,5 Gew.%, bezogen auf das Präpolymer, zugegeben.

Werden die Verbindungen der Formel I als Härter eingesetzt, so soll das Mol-Verhältnis von freigesetzten >NH-Gruppen zu freien Isocyanatgruppen im Präpolymer 0,5 bis 1,5:1; vorzugsweise 0,9 bis 1,1:1 betragen.

Beispiel 1:

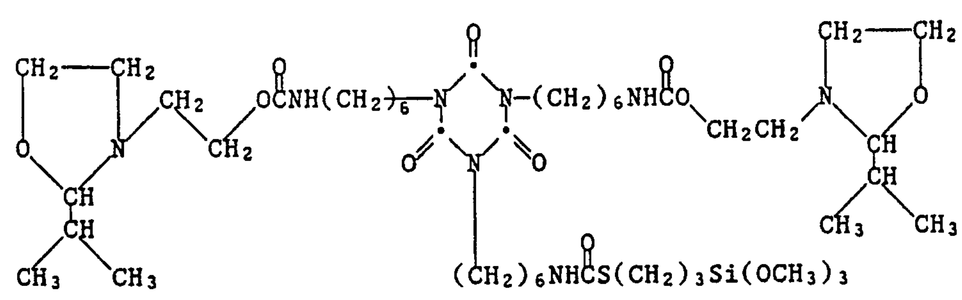

Eine Mischung aus 50 g partiell trimerisiertem Hexamethylendiisocyanat mit einem Isocyanatgehalt von 21,6 % (Desmodur® N3200 der Firma Bayer AG) und 16,8 g 3-Mercaptopropyltrimethoxysilan wird 60 Minuten bei 140 °C erhitzt bis ein Isocyanatgehalt von 10,4 % erreicht ist. Dann lässt man auf 100 °C

abkühlen und gibt eine Mischung aus 27,2 g N-(2-Hydroxyethyl)-2-isopropyliden-(1,3)-oxazolidin (Kp 54°C/13,3 Pa; hergestellt aus Isobutyraldehyd und Diethanolamin) und 100 ml trockenem Toluol zum Reaktionsgemisch und rührt 1 Stunde bei 100°C bis kein freies Isocyanat mehr nachweisbar ist. Danach wird das Toluol bei 95°C/17,3 kPa am Rotationsverdampfer abgezogen und man erhält 93 g (98 %) eines vikosen Harzes mit den folgenden Analysendaten:
Viskosität (nach Epprecht): $\eta_{40}$ = 181 760 mPa•s
Amingehalt (Titration): 1,73 Mol $NR_3$/kg (ber. 1,96)

| Elementaranalyse: gefunden berechnet für $C_{46}H_{86}N_8O_{13}SSi$ | | |
|---|---|---|
| % C | 54,80 | 54,36 |
| % H | 8,74 | 8,74 |
| % N | 11,90 | 11,03 |
| % S | 2,98 | 3,12 |

Beispiel 2:

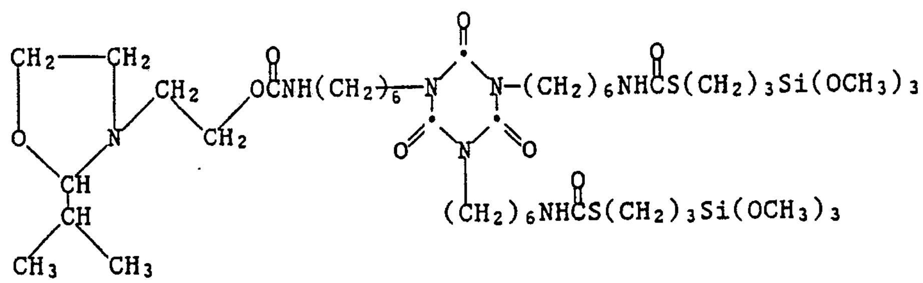

Eine Mischung aus 45 g partiell trimerisiertem Hexamethylendiisocyanat mit einem Isocyanatgehalt von 21,6 % (Desmodur® N3200 der Firma Bayer AG) und 30,3 g 3-Mercaptopropyltrimethoxysilan wird 60 Minuten bei 140°C gerührt bis ein Isocyanatgehalt von 4,0 % erreicht ist. Dann lässt man auf 100°C abkühlen und gibt eine Mischung aus 12,2 g N-(2-Hydroxyethyl) 2-isopropyliden-(1,3)-oxazolidin (Kp 54°C/13,3 Pa; hergestellt aus Isobutyraldehyd und Diethanolamin) und 100 ml trockenem Toluol zum Reaktionsgemisch und rührt 1 Stunde bei 100°C bis kein freies Isocyanat mehr nachweisbar ist. Danach wird das Toluol bei 95°C/17,3 kPa am Rotationsverdampfer abgezogen und man erhält 83,5 g (95 %) eines viskosen Harzes mit den folgenden Analysendaten:
Viskosität (nach Epprecht) $\eta_{40}$ = 353 280 mPa•s
Amingehalt (Titration): 0,81 Mol $NR_3$/kg (berechnet 0,94)

| Elementaranalyse: gefunden berechnet für $C_{44}H_{85}N_7O_{14}S_2Si_2$ | | |
|---|---|---|
| % C | 50,75 | 50,02 |
| % H | 8,74 | 8,11 |
| % N | 10,31 | 9,28 |
| % S | 5,85 | 6,07 |

Beispiel 3:

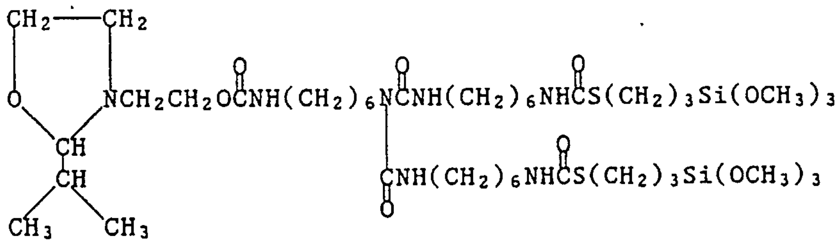

Eine Mischung aus 50 g biurethaltigem partiell hydrolysiertem Hexamethylendiisocyanat mit einem Isocyanatgehalt von 21,3 % (Desmodur® N100 der Firma Bayer AG) und 33,5 g 3-Mercaptopropyltrimethoxysilan wird 60 Minuten bei 140° C erhitzt bis ein Isocyanatgehalt von 4,5 % erreicht ist. Dann lässt man auf 100° C abkühlen und gibt 13,5 g N-(2-Hydroxyethyl)-2-isopropyliden-(1,3)oxazolidin (Kp 54° C/13,3 Pa; hergestellt am Isobutyraldehyd und Diethanolamin) und 100 ml trockenes Toluol zu und rührt 1 Stunde bei 100° C bis kein freies Isocyanat mehr nachweisbar ist. Danach wird das Toluol bei 95° C/17,3 kPa am Rotationsverdampfer abgezogen und man erhält 95,3 g (98 %) eines viskosen Harzes mit den folgenden Analysendaten.
Viskosität (nach Epprecht) $\eta_{40}$ = 179 800 mPa•s
Amingehalt (Titration): 0,93 Mol $NR_3$/kg (berechnet 0,97)

| Elementaranalyse: gefunden berechnet für $C_{43}H_{87}N_7O_{13}S_2Si_2$ | | |
|---|---|---|
| % C | 50,65 | 50,12 |
| % H | 8,50 | 8,51 |
| % N | 10,39 | 9,51 |
| % S | 5,64 | 6,22 |

Beispiel 4:

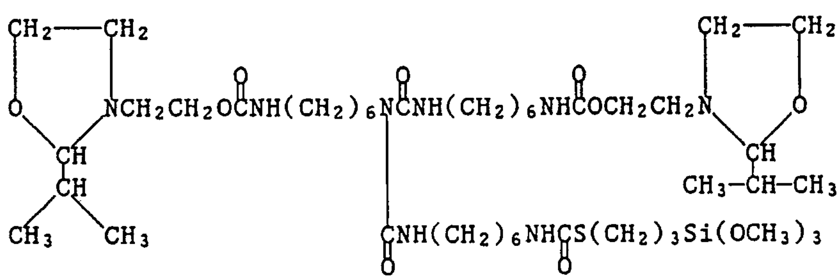

Eine Mischung aus 50 g biurethaltigem partiell hydrolysiertem Hexamethylendiisocyanat mit einem Isocyanatgehalt von 21,3 % (Desmodur® N100 der Firma Bayer AG) und 16,7 g 3-Mercaptopropyltrimethoxysilan wird 1 Stunde bei 140° C erhitzt bis ein Isocyanatgehalt von 10,2 % erreicht ist. Dann gibt man eine Mischung aus 27,0 g N-(2-Hydroxyethyl)-2-isopropyliden-(1,3)-oxazolidin (Kp 54° C/13,3 Pa; hergestellt aus Isobutyraldehyd und Diethanolamin) und 100 ml trockenem Toluol zu und rührt 1 Stunden bei 100° C is kein freies Isocyanat mehr nachweisbar ist. Danach entfernt man das Toluol bei 95° C/17,3 kPa am Rotationsverdampfer und erhält 91,0 g (97 %) eines viskosen Harzes mit den folgenden Analysendaten:
Viskosität (nach Epprecht) $\eta_{40}$ = 322 360 mPa•s
Amingehalt (Titration): 1,94 Mol $NR_3$/kg (berechnet 2,02).

| Elementaranalyse: gefunden berechnet für $C_{45}H_{88}N_8O_{12}SSi$ | | |
|---|---|---|
| % C | 54,81 | 54,44 |
| % H | 8,82 | 8,87 |
| % N | 11,93 | 11,29 |
| % S | 2,97 | 3,22 |

Beispiel 5:

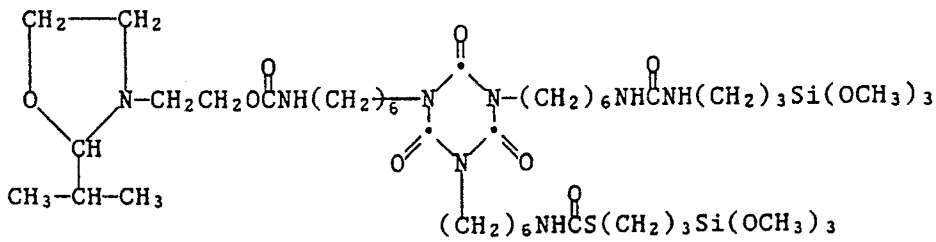

Zu 50 g partiell trimerisiertem Hexamethylendiisocyanat mit einem Isocyanatgehalt von 21,6 % (Desmodur® N3200 der Firma Bayer AG) gibt man 16,8 g 3-Mercaptopropyltrimethoxysilan und erhitzt 60 Minuten bei 140 °C bis ein Isocyanatgehalt von 10,7 % erreicht ist. Dann kühlt man auf 100 °C ab und tropft 13,6 g N-(2-Hydroxyethyl)-2-isopropyliden-(1,3)-oxazolidin (Kp 54 °C/13,3 Pa; hergestellt aus Isobutyraldehyd und Diethanolamin) zu und rührt 30 Minuten bei 100 °C bis ein Isocyanatgehalt von 2 % erreicht ist. Nach Abkühlen auf 40 °C setzt man 15,3 g Aminopropyltrimethoxysilan zu und rührt eine Stunde bis kein Isocyanat mehr nachweisbar ist. Man erhält ein viskoses isocyanatfreies Harz mit den folgenden Analysendaten:

Viskosität (nach Epprecht) $\eta_{80}$ = 5760 mPa•s

Amingehalt (Titration): 0,90 Mol $NR_3$/kg (berechnet 0,96 Mol $NR_3$/kg)

| Elementaranalyse: gefunden berechnet für $C_{44}H_{86}N_8O_{14}SSi_2$ | | |
|---|---|---|
| % C | 51,21 | 50,87 |
| % H | 8,70 | 8,29 |
| % N | 11,38 | 10,80 |
| % S | 2,56 | 3,08 |

Beispiel 6:

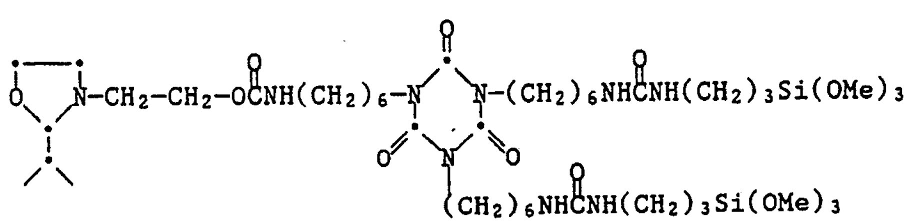

Zu 50 g partiell trimerisiertem Hexamethylendiisocyanat mit einem Isocyanatgehalt von 21,6 % (Desmodur® N3200 der Firma Bayer AG) gibt man 13,6 g N-(2-Hydroxyethyl)-2-isopropyliden-(1,3)-oxazolidin (Kp 54 °C/13,3 Pa; hergestellt aus Isobutyraldehyd und Diethanolamin) und rührt 60 Minuten bei 100 °C bis ein Isocyanatgehalt von 9 % erreicht ist. Dann lässt man abkühlen und gibt bei ca. 30 °C 30,6 g 3-Aminopropyltrimethoxysilan zu und rührt eine Stunde bis kein Isocyanat mehr nachweisbar ist. Man erhält ein viskoses isocyanatfreies Harz mit den folgenden Analysendaten:
Viskosität (nach Epprecht) $\eta_{80}$ = 18080 mPa•s
Amingehalt (Titration): 0,92 Mol $NR_3$/kg (berechnet 0,97 Mol $NR_3$/kg)

| Elementaranalyse: gefunden berechnet für $C_{44}H_{87}N_9O_{14}Si_2$ | | |
|---|---|---|
| % C | 52,47 | 51,71 |
| % H | 8,90 | 8,52 |
| % N | 12,85 | 12,34 |

Beispiel 7:

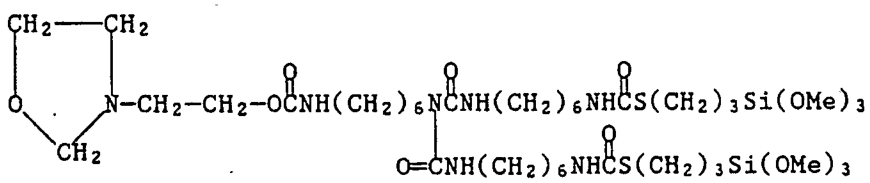

Eine Mischung aus 156 g biurethaltigem partiell hydrolysiertem Hexamethylendiisocyanat mit einem Isocyanatgehalt von 21,3 % (Desmodur® N 100 der Firma Bayer AG) und 110 g 3-Mercaptopropyltrimethoxysilan wird 1 Stunde bei 140 °C erhitzt bis ein Isocyanatgehalt von 2,8 % erreicht ist. Dann lässt man auf 100 °C abkühlen und gibt 31 g N-(2-Hydroxyethyl)-(1,3)-oxazolidin (Kp 99 °C/700 Pa; hergestellt aus Paraformaldehyd und Diethanolamin) und 100 ml trockenes Toluol zu und rührt 1 Stunde bei 100 °C bis kein freies Isocyanat mehr nachweisbar ist. Danach wird das Toluol bei 95 °C/17,3 kPa am Rotationsverdampfer abgezogen und man erhält 290 g (97 %) eines viskosen Harzes mit den folgenden Analysendaten.
Viskosität (nach Epprecht) $\eta_{40}$ = 197120 mPa•s
Amingehalt (Titration): 0,89 Mol $NR_3$/kg (berechnet 1,00)

| Elementaranalyse: gefunden berechnet für $C_{40}H_{81}N_7O_{13}S_2Si_2$ | | |
|---|---|---|
| % C | 49,22 | 48,61 |
| % H | 8,18 | 8,26 |
| % N | 10,51 | 9,92 |
| % S | 6,04 | 6,49 |

Beispiel 8:

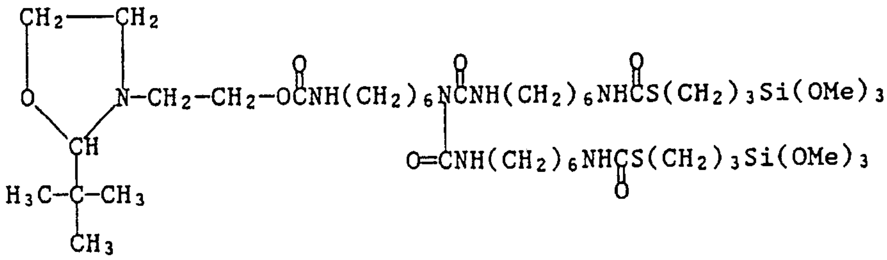

Eine Mischung aus 160,6 g biurethaltigem partiell hydrolysiertem Hexamethylendiisocyanat mit einem Isocyanatgehalt von 21,3 % (Desmodur® N 100 der Firma Bayer AG) und 107,6 g 3-Mercaptopropyltrimethoxysilan wird 1 Stunde bei 140 °C erhitzt bis ein Isocyanatgehalt von 2,8 % erreicht ist. Dann lässt man auf 100 °C abkühlen und gibt 47,3 g N-(2-Hydroxyethyl)-2-(t-butyl)-(1,3)-oxazolidin (Kp 112 °C/700 Pa; hergestellt aus Pivalaldehyd und Diethanolamin) und 100 ml trockenes Toluol zu und rührt 1 Stunde bei 100 °C bis kein freies Isocyanat mehr nachweisbar ist. Danach wird das Toluol bei 95 °C/17,3 kPa am Rotationsverdampfer abgezogen und man erhält 289 g (92 %) eines viskosen Harzes mit den folgenden Analysendaten.

Viskosität (nach Epprecht) $\eta_{80}$ = 6720 mPa•s

Amingehalt (Titration): 0,83 mol $NR_3$/kg (berechnet 0,95)

| Elementaranalyse: gefunden berechnet für $C_{44}H_{88}N_7O_{13}S_2Si_2$ | | |
|---|---|---|
| % C | 51,16 | 50,64 |
| % H | 8,51 | 8,50 |
| % N | 10,21 | 9,40 |
| % S | 5,72 | 6,14 |

Beispiel 9:

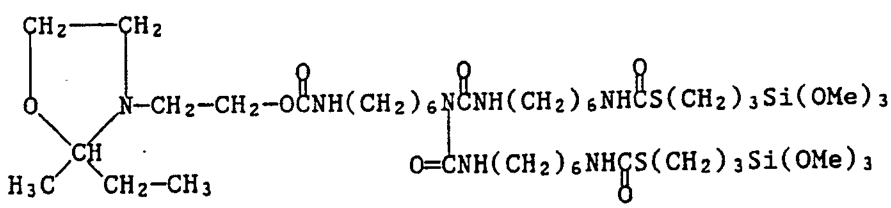

Eine Mischung aus 200 g biurethaltigem partiell hydrolysiertem Hexamethylendiisocyanat mit einem Isocyanatgehalt von 21,3 % (Desmodur® N 100 der Firma Bayer AG) und 134 g 3-Mercaptopropyltrimethoxysilan wird 1 Stunde bei 140 °C erhitzt bis ein Isocyanatgehalt von 2,8 erreicht ist. Dann lässt man auf 100 °C abkühlen und gibt 54,1 g N-(2-Hydroxyethyl)-2-methyl-2-ethyl-(1,3)-oxazolidin (Kp 114 °C/500 Pa; hergestellt aus Methylethylketon und Diethanolamin) und 100 ml trockenes Toluol zu und rührt 1 Stunde bei 100 °C bis kein freies Isocyanat mehr nachweisbar ist. Danach wird das Toluol bei 95 °C/17,3 kPa am Rotationsverdampfer abgezogen und man erhält 380 g (98 %) eines viskosen Harzes mit den folgenden Analysendaten.

Viskosität (nach Epprecht) $\eta_{80}$ = 18560 mPa•s

Amingehalt (Titration): 0,88 mol $NR_3$/kg (berechnet 0,97)

| Elementaranalyse: gefunden berechnet für $C_{43}H_{87}N_7O_{13}S_2Si_2$ | | |
|---|---|---|
| % C | 50,63 | 50,12 |
| % H | 8,58 | 8,51 |
| % N | 10,26 | 9,51 |
| % S | 5,67 | 6,22 |

Beispiel 10:

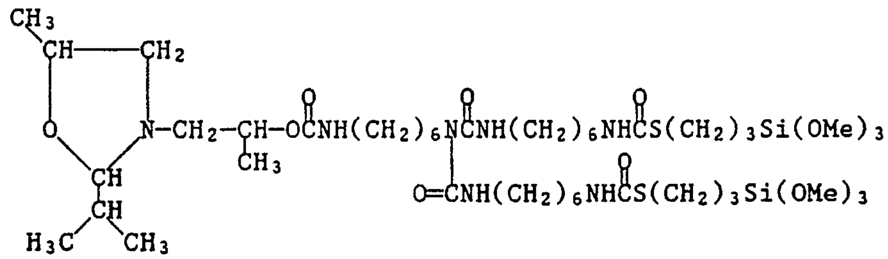

Eine Mischung aus 200 g biurethaltigem partiell hydrolysiertem Hexamethylendiisocyanat mit einem Isocyanatgehalt von 21,3 % (Desmodur® N 100 der Firma Bayer AG) und 141 g 3-Mercaptopropyltrimethoxysilan wird 1 Stunde bei 140° C erhitzt bis ein Isocyanatgehalt von 2,8 % erreicht ist. Dann lässt man auf 100° C abkühlen und gibt 63,5 g N-(2-Hydroxy-2-methylethyl)-2-isopropyliden-4-methyl-(1,3)-oxazolidin (Kp 103° C/700 Pa; hergestellt aus Isobutyraldehyd und Bis-(2-hydroxypropyl)amin) und 100 ml trockenes Toluol zu und rührt 1 Stunde bei 100° C bis kein freies Isocyanat mehr nachweisbar ist. Danach wird das Toluol bei 95° C/17,3 kPa am Rotationsverdampfer abgezogen und man erhält 400 g (98 %) eines viskosen Harzes mit den folgenden Analysendaten.
Viskosität (nach Epprecht) $\eta_{40}$ = 55680 mPa•s
Amingehalt (Titration): 0,86 mol $NR_3$/kg (berechnet 0,94)

| Elementaranalyse: gefunden berechnet für $C_{45}H_{91}N_7O_{13}S_2Si_2$ | | |
|---|---|---|
| % C | 51,19 | 51,06 |
| % H | 8,59 | 8,67 |
| % N | 9,91 | 9,26 |
| % S | 6,04 | 6,06 |

Beispiel 11: Oxazolidin- und Mercaptosilan-terminiertes Präpolymer.

Zu 100 g partiell trimerisiertem Hexamethylendiisocyanat mit einem Isocyanatgehalt von 21,6 % (Desmodur® N3200 der Firma Bayer AG) werden 50 g trockenes bishydroxylterminiertes Polypropylenglykol mit dem Molekulargewicht 1000 (Desmophen® 1600 U der Firma Bayer AG) bei 100° C zugefügt. Dann wird bei 140° C 2 Std. gerührt bis ein Isocyanatgehalt von 11,5 % erreicht ist. Zu dieser Mischung gibt man 53,8 g 3-Mercaptopropyltrimethoxysilan und rührt bei 140° C bis nach 1 Std. ein Isocyanatgehalt von 2,5 % erreicht ist. Dazu werden nun 21,8 g N-(2-hydroxyethyl)-2-isopropyliden-(1,3)-oxazolidin gegeben, worauf die Mischung bei 140° C zwei Stunden gerührt wird bis kein freies Isocyanat mehr nachweisbar ist.

Viskosität (nach Epprecht): $\eta_{40}$ 373 760 mPa•s
Amingehalt (Titration): 0,697 Mol $NR_3$/kg.

Beispiel 12:

A) Präpolymere-Synthese:

Eine isocyanatterminiertes Präpolymer wird hergestellt, indem man bei 80 °C zu 150 g Methylendiphenyldiisocyanat (Isonate® 125M der Firma Upjohn) innerhalb 1 Stunde ein Gemisch aus 531 g trockenem bishydroxylterminiertem Polypropylenglykol mit dem Molekulargewicht 2000 (Desmophen® 1900U der Firma Bayer AG) und 0,3 ml Dibutylzinndilaurat zufliessen lässt. Dann versetzt man mit 2,7 g Trimethylolpropan und rührt weitere 2 Stunden bei 80 °C bis sich ein isocyanatterminiertes Präpolymer mit einem Isocyanatgehalt von 2,7 % gebildet hat.

B) Haftung auf Glas:

Zu 137 g dieses Präpolymers gibt man 6,8 g trockene pyrogene Kieselsäure (Aerosil 380) und Oxazolidinsilan-Haftvermittler gemäss Tabelle 1. Dann giesst man auf eine Glasplatte eine 4 mm dicke Polyurethanschicht. Nach zwei Wochen lagert man diese Proben zwei Wochen in Wasser bei Raumtemperatur. Die Ergebnisse sind in Tabelle 1 zusammengestellt, wobei (-) bedeutet, dass sich die Polyurethanschicht völlig abgetrennt hat, (+) bedeutet, dass sich die Polyurethanschicht nicht abtrennt und beim Abziehen < 50 % Kohäsionsbruch zeigt. (+ +) bedeutet, dass sich die Polyurethanschicht nicht ablöst und beim Abziehen 100 % Kohäsionsbruch auftritt. Ausserdem wird der Aufbau der Shore A-Härte nach DIN 53505 gemessen (Tabelle 2).

Tabelle 1

| Beispiel | Haftvermittler aus Beispiel Nr. | Haftvermittler-Gehalt [Gew.-%] | Haftung auf Glas |
|---|---|---|---|
| | - | - | - |
| A | 1 | 9,0 | + |
| B | 2 | 9,0 | + |
| C | 3 | 4,5 | + + |
| D | 4 | 4,5 | + + |
| E | 5 | 4,5 | + |
| F | 6 | 4,5 | + + |
| J | 7 | 4,5 | + + |
| K | 8 | 4,5 | + + |
| L | 9 | 4,5 | + + |
| M | 10 | 4,5 | + + |

Tabelle 2

| Beispiel | Haftvermittler aus Beispiel Nr. | Haftvermittler-Gehalt [Gew.-%] | Aufbau der Shore-Härte A | | | | |
|---|---|---|---|---|---|---|---|
| | | | 1 | 2 | 5 | 9 | 14 [Tage] |
| | - | - | 1 | 6 | 19 | 29 | 30 |
| G | 3 | 4,5 | 1 | 8,5 | 32 | 32 | 32 |
| H | 4 | 4,5 | 1 | 6 | 32 | 32 | 32 |

**Ansprüche**

1. Verbindungen der allgemeinen Formel I

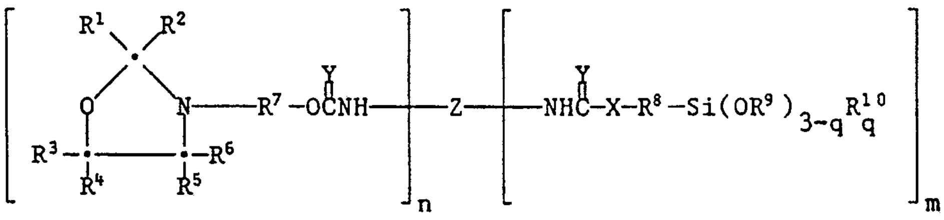

(I)

worin
$R^1$ Wasserstoff, $C_1$-$C_{12}$-Alkyl, $C_5$-$C_7$-Cycloalkyl, Phenyl oder Benzyl ist und
$R^2$ Wasserstoff oder $C_1$-$C_4$-Alkyl bedeutet, oder
$R^1$ und $R^2$ zusammen mit dem C-Atom, an welches sie gebunden sind, einen 5- oder 6-gliedrigen Ring bilden, und $R^3$, $R^4$, $R^5$ und $R^6$ gleich oder verschieden sind und Wasserstoff, $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder eine Gruppe der Formel $-CH_2OR^{11}$ bedeuten, wobei $R^{11}$ $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder -C(O)-$R^{12}$ ist, und $R^{12}$ $C_1$-$C_{12}$-Alkyl bedeutet, und
$R^7$ $C_1$-$C_4$-Alkylen, $R^8$ $C_1$-$C_8$-Alkylen, $R^9$ $C_1$-$C_4$-Alkyl oder zwei Reste $R^9$ zusammen $C_1$-$C_4$-Alkylen, sowie $R^{10}$ $C_1$-$C_4$-Alkyl oder Phenyl darstellen, wobei q die Werte 0 bis 2 bedeutet, ferner X -S- oder -NH- ist, Y = O oder = S ist, und Z ein organischer Rest ist, der sich von einem Polyisocyanat oder einem Polyisothiocyanat mit mindestens 3 NCO- resp. NCS-Gruppen ableitet, und weiter n einen Wert $\geqq 1$ und m einen Wert $\geqq 1$ bedeuten, mit der Massgabe, dass $n + m \geqq 3$ ist.

2. Verbindungen gemäss Anspruch 1, worin Y = O bedeutet.

3. Verbindungen gemäss Anspruch 1 der Formel I, worin Z von einem aliphatischen, cycloaliphatischen, aliphatisch/aromatischen, aromatischen oder heterocyclischen Polyisocyanat oder Polyisothiocyanat mit $\geqq 3$ NCO-oder NCS-Gruppen abgeleitet ist, wobei dieser Rest gegebenenfalls eine oder mehrere Ester-, Ether-, Urethan-, Thiourethan-, Isocyanurat-, Harnstoff- oder Biuret-Funktionen enthalten kann.

4. Verbindungen gemäss Anspruch 3 der Formel I, worin Z von einem aliphatischen oder gemischt aliphatisch/aromatischen Polyisocyanat mit $\geqq 3$ NCO-Gruppen abgeleitet ist, wobei dieser Rest Z gegebenenfalls eine oder zwei Ester-, Ether-, Urethan, Thiourethan, Isocyanurat-, Harnstoff oder Biuret-Funktionen enthält.

5. Verbindungen gemäss Anspruch 1 der Formel I, worin der Rest Z ein mittleres Molekulargewicht $M_n$ < 10000 aufweist.

6. Verbindungen gemäss Anspruch 1, der Formel I, worin n und m unabhängig voneinander die Werte 1 bis 49 besitzen.

7. Verbindungen gemäss Anspruch 1 der Formel I, worin die Summe n + m 3 bis 50 beträgt.

8. Verbindungen gemäss Anspruch 1 der Formel I, worin n die Werte 1 oder 2, und m die Werte 2 oder 1 haben.

9. Verbindungen gemäss Anspruch 1 der Formel I, worin $R^1$ und $R^2$ unabhängig voneinander Wasserstoff oder $C_1$-$C_4$-Alkyl bedeuten.

10. Verbindungen gemäss Anspruch 1 der Formel I, worin $R^3$, $R^4$, $R^5$ und $R^6$ Wasserstoff oder $C_1$-$C_4$-Alkyl und höchstens zwei dieser Reste Phenoxymethyl bedeuten.

11. Verbindungen gemäss Anspruch 1 der Formel I, worin $R^3$ und $R^6$ Wasserstoff und $R^4$ und $R^5$ Wasserstoff oder Methyl bedeuten.

12. Verbindungen gemäss Anspruch 1 der Formel I, worin $R^1$ $C_1$-$C_4$-Alkyl, $R^2$ Wasserstoff, $R^3$, $R^4$, $R^5$ und $R^6$ je Wasserstoff und $R^7$ Ethylen bedeuten.

13. Verbindungen gemäss Anspruch 1 der Formel I, worin X -S-, $R^8$ $C_1$-$C_4$-Alkylen und $R^9$ $C_1$-$C_4$-Alkyl bedeuten und q den Wert 0 hat.

14. Feuchtigkeitshärtende Polyurethanharze, enthaltend mindestens eine Verbindung der Formel I gemäss Anspruch 1.

15. Harze gemäss Anspruch 14, enthaltend 0,1-20 Gew.-% einer Verbindung der Formel I.

Patentansprüche für folgenden Vertragsstaat: ES

1. Verfahren zur Herstellung von Verbindungen der allgemeinen Formel I

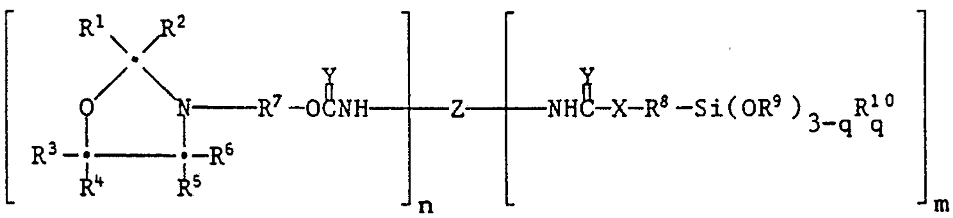 (I)

worin
$R^1$ Wasserstoff, $C_1$-$C_{12}$-Alkyl, $C_5$-$C_7$-Cycloalkyl, Phenyl oder Benzyl ist und
$R^2$ Wasserstoff oder $C_1$-$C_4$-Alkyl bedeutet, oder
$R^1$ und $R^2$ zusammen mit dem C-Atom, an welches sie gebunden sind, einen 5- oder 6-gliedrigen Ring bilden, und $R^3$, $R^4$, $R^5$ und $R^6$ gleich oder verschieden sind und Wasserstoff, $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder eine Gruppe der Formel $-CH_2OR^{11}$ bedeuten, wobei $R^{11}$ $C_1$-$C_{12}$-Alkyl, unsubstituiertes oder mit 1-3 $C_1$-$C_4$-Alkyl, Halogen oder $C_1$-$C_4$-Alkoxy substituiertes Phenyl oder $-C(O)-R^{12}$ ist, und $R^{12}$ $C_1$-$C_{12}$-Alkyl bedeutet, und
$R^7$ $C_1$-$C_4$-Alkylen, $R^8$ $C_1$-$C_8$-Alkylen, $R^9$ $C_1$-$C_4$-Alkyl oder zwei Reste $R^9$ zusammen $C_1$-$C_4$-Alkylen, sowie $R^{10}$ $C_1$-$C_4$-Alkyl oder Phenyl darstellen, wobei q die Werte 0 bis 2 bedeutet, ferner X -S- oder -NH- ist, Y = O oder = S ist, und Z ein organischer Rest ist, der sich von einem Polyisocyanat oder einem Polyisothiocyanat mit mindestens 3 NCO- resp. NCS-Gruppen ableitet, und weiter n einen Wert $\geqq 1$ und m einen Wert $\geqq 1$ bedeuten, mit der Massgabe, dass $n + m \geqq 3$ ist, durch Umsetzung von n Molen eines Oxazolidins der nachstehenden Formel,

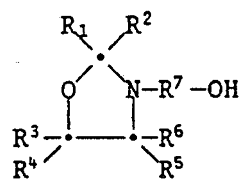

worin die Reste $R^1$ bis $R^7$ die vorstehende Bedeutung haben, und m Molen eines Silans der allgemeinen Formel
$HX-R^8-Si(OR^9)_{3-q}R^{10}_q$
worin $R^8$, $R^9$, $R^{10}$, X und q die vorstehend gegebene Bedeutung haben mit einem Polyisocyanat oder einem Polyisothiocyanat der Formeln
$Z(NCO)_{\geq 3}$ oder $Z(NCS)_{\geq 3}$.

2. Verfahren gemäss Anspruch 1, worin Y = O bedeutet.

3. Verfahren gemäss Anspruch 1, worin Z von einem aliphatischen, cycloaliphatischen, aliphatisch/aromatischen, aromatischen oder heterocyclischen Polyisocyanat oder Polyisothiocyanat mit $\geqq 3$ NCO-oder NCS-Gruppen abgeleitet ist, wobei dieser Rest gegebenenfalls eine oder mehrere Ester-, Ether-, Urethan-, Thiourethan-, Isocyanurat-, Harnstoff-oder Biuret-Funktionen enthalten kann.

4. Verfahren gemäss Anspruch 3, worin Z von einem aliphatischen oder gemischt aliphatisch/aromatischen Polyisocyanat mit $\geqq 3$ NCO-Gruppen abgeleitet ist, wobei dieser Rest Z gegebenenfalls eine oder zwei Ester-, Ether-, Urethan, Thiourethan, Isocyanurat-, Harnstoff oder Biuret-Funktionen enthält.

5. Verfahren gemäss Anspruch 1, worin der Rest Z ein mittleres Molekulargewicht $M_n < 10000$ aufweist.

6. Verfahren gemäss Anspruch 1, worin n und m unabhängig voneinander die Werte 1 bis 49 besitzen.

7. Verfahren gemäss Anspruch 1, worin die Summe n + m 3 bis 50 beträgt.

8. Verfahren gemäss Anspruch 1, worin n die Werte 1 oder 2, und m die Werte 2 oder 1 haben.

9. Verfahren gemäss Anspruch 1, worin $R^1$ und $R^2$ unabhängig voneinander Wasserstoff oder $C_1$-$C_4$-Alkyl bedeuten.

10. Verfahren gemäss Anspruch 1, worin $R^3$, $R^4$, $R^5$ und $R^6$ Wasserstoff oder $C_1$-$C_4$-Alkyl und höchstens zwei dieser Reste Phenoxymethyl bedeuten.

11. Verfahren gemäss Anspruch 1, worin $R^3$ und $R^6$ Wasserstoff und $R^4$ und $R^5$ Wasserstoff oder Methyl bedeuten.

12. Verfahren gemäss Anspruch 1, worin $R^1$ $C_1$-$C_4$-Alkyl, $R^2$ Wasserstoff, $R^3$, $R^4$, $R^5$ und $R^6$ je Wasserstoff und $R^7$ Ethylen bedeuten.

13. Verfahren gemäss Anspruch 1, worin X -S-, $R^8$ $C_1$-$C_4$-Alkylen und $R^9$ $C_1$-$C_4$-Alkyl bedeuten und q den Wert 0 hat.

14. Feuchtigkeitshärtende Polyurethanharze, enthaltend mindestens eine Verbindung der Formel I gemäss Anspruch 1.

15. Harz gemäss Anspruch 14, enthaltend 0,1-20 Gew.-% einer Verbindung der Formel I.